# EUROPÄISCHE PATENTSCHRIFT

(11) **EP 2 619 173 B1**
(45) Veröffentlichungstag und Bekanntmachung des Hinweises auf die Patenterteilung: **21.06.2017**
(21) Anmeldenummer: 11758207.2
(22) Anmeldetag: 21.09.2011
(51) Int. Cl.: C07C 213/06, C07C 219/08

(54) **VERFAHREN ZUR HERSTELLUNG VON (METH)ACRYLSÄUREESTERN VON N,N-SUBSTITUIERTEN AMINOALKOHOLEN**
PROCESS FOR PREPARING (METH)ACRYLIC ESTERS OF N,N-SUBSTITUTED AMINO ALCOHOLS
PROCÉDÉ DE PRODUCTION D'ESTERS D'ACIDES (MÉTH)ACRYLIQUES D'ALCOOLS AMINÉS À SUBSTITUTION N,N

(30) Priorität: 23.09.2010 EP 10178757
(43) Veröffentlichungstag der Anmeldung: 31.07.2013
(73) Patentinhaber: BASF SE, 67056 Ludwigshafen am Rhein (DE)
(72) Erfinder: BETTE, Virginie, 68199 Mannheim (DE); BERGMANN, Hermann, Singapore 259283 (SG); PETZOLDT, Jochen, 67273 Weisenheim am Berg (DE); HÖFER, Frank, 67098 Bad Dürkheim (DE)
(74) Vertreter: BASF IP Association
(86) Internationale Anmeldenummer: PCT/EP2011/066386
(87) Internationale Veröffentlichungsnummer: WO 2012/038459

(56) Entgegenhaltungen:
- DE-A1- 3 423 441

## Beschreibung

Die vorliegende Erfindung betrifft ein Verfahren zur katalytischen Herstellung von (Meth)acrylsäureestern von N,N-substituierten Aminoalkoholen und deren Verwendung.

Unter (Meth)acrylsäure im Sinne der vorliegenden Erfindung werden Acrylsäure und/oder Methacrylsäure verstanden, unter (Meth)acrylsäureester Acrylsäureester und/oder Methacrylsäureester. Im Folgenden werden (Meth)acrylsäureester auch als (Meth)acrylate bezeichnet.

Die Herstellung von (Meth)acrylsäureestern erfolgt zumeist durch säure- oder basenkatalysierte Veresterung von (Meth)acrylsäure oder Umesterung von anderen (Meth)acrylsäureestern mit Alkoholen. Dabei werden häufig starke Säuren oder Basen eingesetzt, so dass sich säure- oder baseempfindliche (Meth)acrylsäureester durch eine Ver- oder Umesterung auf diesem Wege nicht gezielt herstellen lassen.

(Meth)acrylsäureester von N,N-substituierten Aminoalkoholen sowie deren Herstellung unter verschiedenen Bedingungen sind bekannt.

So offenbart EP 1 399 408 A1 die Umesterung zur Herstellung von N,N-Dialkylaminoalkyl-(meth)acrylaten unter Verwendung von Titanalkoholaten als Katalsysatoren. Das Reinprodukt wird nach der Reaktion durch Destillation erhalten.

In EP 0 118 639 A1 wird die Umesterung zur Herstellung von N,N-Dialkylaminoalkyl(meth)-acrylaten unter Verwendung von Ti-, Al-, Zr-, Ca- und Mg-Alkoholaten beschrieben.

Die DE 3423441 offenbart laut Beschreibung die Herstellung von (Meth)acrylsäureestern durch Umesterung von (Meth)acrylsäureestern von C1- bis C4-Alkoholen unter Verwendung eines Li-Ca-Katalysatorsystems. Die zur Umesterung verwendeten Alkohole sind zu den C1- bis C4-Alkohlen verschieden und umfassen keine mehrwertigen Alkohole.

Aus JP 2001187763 ist die Verwendung von organischen Zinnoxiden als Katalysator für die Umesterungsreaktion von Alkyl(meth)acrylaten mit verschiedenen Alkoholen wie beispielsweise N,N-Dimethylaminoethanol und N,N-Diethylaminoethanol beschrieben. Der Einsatz von Dibutylzinnoxid als Katalysator für die Umesterungsreaktion zur Herstellung von N,N-Dialkylamino-alkyl(meth)acrylaten ist ebenfalls aus EP 0 906 902 A1 bekannt. Dabei wird der Katalysator abschließend vom Reaktionsgemisch destillativ abgetrennt.

Weiterhin offenbart die WO 03/022796 A1 die Verwendung von Zirkoniumacetylacetat als Katalysator für die Synthese von N,N-Dialkylaminoalkyl(meth)acrylaten aus N,N-Dialkylaminoalkoholen und Methylmethacrylat. Die Abtrennung des Zr-haltigen Katalysators erfolgt ebenfalls destillativ.

Darüber hinaus offenbaren JPH0651664 und JPS62230755 die Verwendung von Kaliumphosphat als Katalysator für die Umesterungsreaktion von (Meth)acrylaten mit verschiedenen N,N-Dialkylaminoalkoholen. Dabei wird der Katalysator in dem jeweiligen N,N-Dialkylaminoalkohol oder alternativ in Methanol gelöst. Gemäß der Lehre dieser Schriften werden geringere Ausbeuten erhalten, wenn der Katalysator als Suspension in die Reaktionsmischung eingebracht wird. Zur Stabilisierung der Reaktionsmischung werden übliche Polymerisationsinhibitoren wie Phenothiazin und Hydrochinonmonomethylether in Mengen von 0,05 - 5 Gew.-% (500 - 20 000 ppm) verwendet. Das Endprodukt wird ebenfalls destillativ vom verbliebenen Katalysator abgetrennt.

Aus JPH0651663 ist ein ähnlicher Prozess bekannt, allerdings wird dort die Verwendung von Kaliumcarbonat als Katalysator beschrieben. Dieser wird zunächst in Methanol gelöst und anschließend zur Reaktionsmischung gegeben. Das Endprodukt wird ebenfalls destillativ aufgereinigt, um den Katalysator nach Reaktionsende abzutrennen. Die im Stand der Technik beschriebenen Verfahren weisen einige Nachteile auf. Die darin offenbarten Katalysatoren sind teilweise feuchtigkeitsempfindlich und relativ teuer, insbesondere für eine industrielle Anwendung (beispielsweise Ti- und Sn- haltige Katalysatoren). Ferner offenbaren alle Dokumente einen aufwendigen Reinigungsschritt, wobei das Endprodukt immer destillativ vom Katalysator abgetrennt werden muss.

Die japanischen Schriften JPH0651664, JPS62230755 und JPH0651663 weisen darüber hinaus den Nachteil auf, dass die Katalysatoren Kaliumphosphat bzw. Kaliumcarbonat zunächst in einem geeigneten Lösungsmittel gelöst werden müssen, bevor sie der Reaktionsmischung zugeführt werden. Dies hat ebenfalls eine destillative Aufreinigung des Endprodukts zur Folge. Ferner wird in allen drei Schriften der Einsatz von Polymerisationsinibitoren in hohen Mengen (> 500 ppm) offenbart.
Aufgabe der vorliegenden Erfindung war es daher, ein weiteres Verfahren zur Verfügung zu stellen, mit dem (Meth)acrylsäureester von N,N-substituierten Aminoalkoholen in hohen Umsätzen und hohen Reinheiten aus einfachen Edukten herstellbar sind. Ferner sollen die oben beschriebenen Nachteile der im Stand der Technik beschriebenen Verfahren überwunden werden, insbesondere soll die Aufreinigung des Endprodukts einfacher sein oder gar ganz entfallen, und ferner soll der Gehalt an Polymerisationsinhibitoren reduziert werden.
Die Aufgabe wurde gelöst durch ein Verfahren zur Herstellung von (Meth)acrylsäureestern (F) von N,N-substituierten Aminoalkoholen, in dem man N,N-substituierten Aminoalkohole (I), 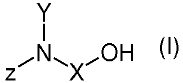 worin
- Y und Z: unabhängig voneinander C₁-C₂₀-Alkyl, C₃-C₁₅-Cycloalkyl, Aryl oder Y und Z zusammen mit dem sie verbindenden Stickstoffatom einen 5- bis 9-gliedrigen gesättigten heterocyclischen Rest bilden, der gegebenenfalls Sauerstoff, Schwefel, Stickstoff oder C₁-C₄-alkylsubstituierten Stickstoff als weiteres Heteroatom aufweist, und
- X: C₂-C₂₀-Alkylen, das durch 1 bis 10, bevorzugt 1 bis 5, insbesondere 1 oder 2 nichtbenachbarte Oxy- und/oder unsubstituierte oder mit oder Methoxy substituierte C₁-C₄-Alkyliminogruppen unterbrochen sein kann, oder C₃-C₁₅-Cycloalkylen
bedeuten,
in Gegenwart mindestens eines heterogenen Katalysators (K) mit mindestens einem (Meth)-acrylsäureester (D) umestert, dadurch gekennzeichnet, dass der heterogene Katalysator (K) ein anorganisches Salz ist, ausgewählt aus der Gruppe bestehend aus Li₃PO₄, K₃PO₄, Na₃PO₄ und K₂CO₃ sowie deren Hydrate und ohne weitere Lösungsmittel eingesetzt wird und der Gehalt an Polymerisationsinhibitoren in der Reaktionsmischung < 450 ppm, bevorzugt <400 ppm beträgt.

Mit Hilfe des erfindungsgemäßen Verfahrens ist die Herstellung von (Meth)acrylsäureestern von N,N-substituierten Aminoalkoholen mit mindestens einem der folgenden Vorteile möglich:
- Verwendung von günstigen Edukten,
- hohe Ausbeute,
- hohe Reinheit,
- keine aufwendige Aufarbeitung (z.B. destillative Abtrennung des Endproduktes oder von Lösungsmitteln, Abtrennung von Wasser).

Besonders vorteilhaft an dem erfindungsgemäßen Verfahren ist der Verzicht von geeigneten Lösungsmitteln für den Katalysator. Der heterogene Katalysator (K) wird in der Reaktionsmischung suspendiert, so dass eine aufwendige Lösungsmittelabtrennung durch Destillation nicht erforderlich ist. Stattdessen kann der heterogene Katalysator (K) durch einfache Filtration vom Endprodukt abgetrennt werden.

Ferner ist vorteilhaft, dass lediglich eine geringe Menge eines Polymerisationsinhibitors eingesetzt wird und dennoch eine ausreichende Polymerisationsinhibierung erzielt wird.

Im Einzelnen haben die für die verschiedenen Reste angegebenen Sammelbegriffe folgende Bedeutung:
C₁-C₂₀-Alkyl: geradkettige oder verzweigte Kohlenwasserstoffreste mit bis zu 20 Kohlenstoffatomen, bevorzugt C₁-C₁₀-Alkyl wie Methyl, Ethyl, Propyl, Isopropyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Octyl, 2-Ethylhexyl, 2,4,4-Trimethylpentyl, 1,1,3,3-Tetramethylbutyl, Nonyl und Decyl sowie deren Isomere.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl und Cyclooctyl sowie ein gesättigtes oder ungesättigte cyclisches System wie z. B. Norbornyl oder Norbenyl.

Aryl: ein ein- bis dreikerniges aromatisches Ringsystem enthaltend 6 bis 14 Kohlenstoffringglieder, z. B. Phenyl, Naphthyl und Anthracenyl, bevorzugt ein ein- bis zweikerniges, besonders bevorzugt ein einkerniges aromatisches Ringsystem.

C₂-C₂₀ Alkylen, das nichtbenachbarte Oxy- und/oder unsubstituierte oder mit oder Methoxy substituierte C₁-C₄-Alkyliminogruppen unterbrochen sein kann: Ethylen, 1,2- oder 1,3- Propylen, 1,2-, 2,3- oder 1,4 -Butylen, (CH₂)₂O(CH₂)₂, (CH₂)₃O(CH₂)₃, (CH₂)₂O(CH₂)₂O(CH₂)₂,

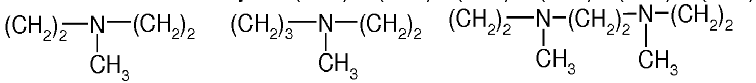

Wenn die Reste Y und Z zusammen mit dem sie verbindenden Stickstoffatom einen 5- bis 9-gliedrigen, bevorzugt 5- oder 6-gliedrigen heterocyclischen Rest bilden, so können gesättigte heterocyclische Reste wie Pyrrolidinyl, Piperidinyl, Morpholinyl, Piperazinyl, in Betracht kommen.

Die Substituenten Y und Z können jeweils gleich oder verschieden sein.

Gemäß einer bevorzugten Ausführungsform bedeuten die Substituenten Y und Z jeweils unabhängig voneinander C₁-C₁₀-Alkyl, Aryl oder Y und Z zusammen mit dem sie verbindenden Stickstoffatom einen 5- bis 9-gliedrigen heterocyclischen Rest, der gegebenenfalls Sauerstoff, Stickstoff oder C₁-C₄-alkylsubstituierten Stickstoff als weiteres Heteroatom aufweist.

Gemäß einer weiteren bevorzugten Ausführungsform bilden die Substituenten Y und Z zusammen mit dem sie verbindenden Stickstoffatom einen 5- oder 6-gliedrigen heterocyclischen Rest, der gegebenenfalls Sauerstoff, Stickstoff oder C₁-C₄-alkylsubstituierten Stickstoff als weiteres Heteroatom aufweist.

In einer weiteren bevorzugten Ausführungsform sind die Substituenten Y und Z jeweils unabhängig voneinander C₁-C₁₀-Alkyl oder Aryl, bevorzugt C₁-C₆-Alkyl oder Phenyl und insbesondere bevorzugt C₁-C₄-Alkyl oder Phenyl, oder bilden gemeinsam einen Heterocyclus, bevorzugt Piperidyl oder Morpholyl.

In einer bevorzugten Ausführungsform steht das Brückenglied X für C₂-C₁₀-Alkylen, das durch 1 oder 5 nichtbenachbarte Oxy- und/oder unsubstituierte oder mit Methoxy substituierte C₁-C₄-Alkyliminogruppen unterbrochen sein kann, insbesondere für C₂-C₁₀-Alkylen, bevorzugt C₂-C₆-Alkylen, und insbesondere bevorzugt C₂-C₄-Alkylen.

Erfindungsgemäß geeignete N,N-substituierte Aminoalkohole (I) sind beispielsweise N,N-Dibutylaminoethanol, N,N-Dimethylaminopropanol, 2-[(2-Dimethylamino-ethyl)-methyl-amino]-ethanol, 2-(2-Dimethylamino-ethoxy)-ethanol, N,N-Diethylaminoethanol, Piperidylethanol, Morpholylethanol und Ethylanilinethanol.

Soweit die N,N-substituierten Aminoalkohole (I) optisch aktiv sind, werden sie bevorzugt racemisch oder als Diastereomerengemisch eingesetzt, es ist jedoch auch möglich, sie als reine Enantiomere bzw. Diastereomere oder als Enantiomerengemische einzusetzen.

Allgemeines Verfahren zur Herstellung von Acrylaten
Im Reaktionsschritt erfolgt die Umesterung des N,N-substituierten Aminoalkohols (I) mit mindestens einem, bevorzugt genau einem (Meth)acrylsäureester (D) erfindungsgemäß in Anwesenheit mindestens eines Katalysators (K).

Zur Umesterung können (Meth)acrylsäureester (D) eines gesättigten Alkohols eingesetzt werden, bevorzugt gesättigte C₁ - C₁₀-Alkylester oder C₃ - C₁₂-Cycloalkylester der (Meth)acrylsäure, besonders bevorzugt gesättigte C₁ - C₄-Alkylester der (Meth)acrylsäure.

Gesättigt bedeutet im Rahmen dieser Schrift Verbindungen ohne C-C-Mehrfachbindungen (außer selbstverständlich die C=C-Doppelbindung in den (Meth)acryleinheiten).

Beispiele für (Meth)acrylsäureester (D) sind (Meth)acrylsäuremethyl-, -ethyl-, -n-butyl-, -iso-butyl-, -tert-butyl-, n-octyl-, -2-ethylhexyl- und cyclohexylester, 1,2-Ethylenglycoldi- und -mono-(meth)acrylat, 1,4-Butandioldi- und -mono(meth)acrylat, 1,6-Hexandioldi- und -mono(meth)acrylat, Trimethylolpropantri(meth)acrylat und Pentaerythrittetra(meth)acrylat.

Besonders bevorzugt sind (Meth)acrylsäuremethyl-, -ethyl-, -n-butyl- und -2-ethyl-hexylester, ganz besonders bevorzugt (Meth)acrylsäuremethyl-, -ethyl- und -n-butylester, insbesondere (Meth)acrylsäuremethyl- und -ethylester und speziell (Meth)acrylsäuremethylester.

Erfindungsgemäß einsetzbare Katalysatoren (K) sind heterogene Katalysatoren, die in der Reaktionsmischung ohne den Einsatz von weiteren Lösungsmitteln suspendiert werden.

Heterogene Katalysatoren sind im Rahmen dieser Schrift solche, die eine Löslichkeit im Reaktionsmedium bei 25 °C von nicht mehr als 1 g/l aufweisen, bevorzugt von nicht mehr als 0,5 g/l und besonders bevorzugt von nicht mehr als 0,25 g/l.

Als Katalysatoren (K) werden anorganische Salze eingesetzt.

Selbstverständlich können die anorganischen Salze in wasserfreier Form oder in Form ihrer Hydrate eingesetzt werden. Bevorzugt werden sie jedoch in wasserfreier Form eingesetzt.

Anorganische Salze sind Li₃PO₄, K₃PO₄, Na₃PO₄, K₂CO₃ und Na₂CO₃ sowie deren Hydrate, bevorzugt ist K₃PO₄.

K₃PO₄ kann erfindungsgemäß in wasserfreier Form eingesetzt werden sowie als Tri-, Hepta- oder Nonahydrat.

Die katalysierte Umesterung erfolgt im Allgemeinen bei 30 bis 140 °C, bevorzugt bei 30 bis 100 °C, besonders bevorzugt bei 40 bis 90 °C und ganz besonders bevorzugt bei 50 bis 80°C.

Gegebenenfalls kann die Reaktion unter leichtem Vakuum von beispielsweise 200 hPa bis Normaldruck, bevorzugt 200 bis 600 hPa und besonders bevorzugt 250 bis 500 hPa durchgeführt werden, wenn der bei der Umesterung entstehende niedrigsiedende Alkohol, gegebenenfalls als Azeotrop, abdestilliert werden soll.

Das molare Verhältnis zwischen (Meth)acrylsäureester (D) und N,N-substituiertem Aminoalkohol (I) beträgt bei der durch einen der oben genannten Katalysatoren (K) katalysierten Umesterung in der Regel 1 : 1 bis 10 : 1 mol/mol, bevorzugt 1 : 1 bis 5 : 1 mol/mol und besonders bevorzugt 1 : 1 bis 4 : 1 mol/mol.

Die Reaktionszeit bei der erfindungsgemäß katalysierten Umesterung beträgt in der Regel 45 min bis 18 Stunden, bevorzugt 2 Stunden bis 12 Stunden und besonders bevorzugt 3 bis 10 Stunden.

Der Gehalt an Katalysator (K) im Reaktionsmedium liegt in der Regel im Bereich von etwa 0,01 bis 10 mol-%, bevorzugt 0,1 - 3,0 und besonders bevorzugt 0,3 - 2,0 mol-% bezogen auf die Summe der eingesetzten N,N-substituierten Aminoalkohole (I).

Bei der Umesterung sind Polymerisationsinhibitoren (wie unten beschrieben) zwingend erforderlich.

Die Anwesenheit von sauerstoffhaltigen Gasen (s.u.) während der Durchführung des erfindungsgemäßen Verfahrens ist bevorzugt.

Bei der erfindungsgemäßen Umesterung werden in der Regel Produkte mit einer Farbzahl unter 500 APHA, bevorzugt unter 200 und besonders bevorzugt unter 150 (gemäß DIN ISO 6271) erhalten.

Die Reaktion kann in organischen Lösungsmitteln oder deren Gemischen oder ohne Zusatz von Lösungsmitteln ablaufen. Die Ansätze sind in der Regel weitgehend wasserfrei (d.h. unter 10, bevorzugt unter 5, besonders bevorzugt unter 1 und ganz besonders bevorzugt unter 0,5 Gew.-% Wassergehalt).

Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethylenglycoldi-C₁-C₄-alkylether, wie z. B. 1,2-Dimethoxyethan, Diethylenglycoldimethylether, Polyethylenglycoldimethylether 500, C₁-C₄-Alkylencarbonate, insbesondere Propylencarbonat, C₃-C₆-Alkylessigsäureester, insbesondere tert.-Butyl-essigsäureester, THF, Toluol, 1,3-Dioxolan, Aceton, iso-Butyl-methylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen.

Bevorzugt wird jedoch auf den Einsatz von Lösungsmitteln verzichtet.

In einer besonders bevorzugten Ausführungsform der Umesterung wird die Reaktion in dem als Edukt eingesetzten (Meth)acrylsäureester (D) durchgeführt. Ganz besonders bevorzugt ist die Durchführung der Reaktion in der Weise, dass das Produkt (F) nach Beendigung der Reaktion als etwa 10 - 80 Gew.-%ige Lösung in dem als Edukt eingesetzten (Meth)acrylsäureester (D) anfällt, insbesondere als 20 bis 50 Gew.-%ige Lösung.

Die Edukte liegen entweder gelöst, als Feststoffe suspendiert oder in Emulsion im Reaktionsmedium vor.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Die Durchmischung kann beispielsweise durch Einspeisen eines Gases, bevorzugt eines sauerstoffhaltigen Gases (siehe unten) erfolgen. Das Reaktionsmedium kann ein- oder mehrphasig sein und die Reaktanden werden darin gelöst, suspendiert oder emulgiert. Die Temperatur wird während der Reaktion auf den gewünschten Wert eingestellt und kann, falls gewünscht, während des Reaktionsverlaufs erhöht oder verringert werden.

Wird die Reaktion im Festbettreaktor durchgeführt, so ist der Festbettreaktor bevorzugt mit immobilisiertem Katalysator (K) bestückt, wobei die Reaktionsmischung durch eine mit dem Katalysator (K) gefüllte Säule gepumpt wird. Es ist auch möglich, die Umsetzung im Wirbelbettreaktor durchzuführen, wobei der Katalysator (K) auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen, wobei auch unter Vakuum der freigesetzte Alkohol gleichzeitig abdestilliert werden kann.

Die Entfernung von Alkoholen, die bei einer Umesterung aus den (Meth)acrylsäureestern (D) freigesetzt werden, erfolgt kontinuierlich oder schrittweise in an sich bekannter Weise, z.B. durch Vakuum, azeotrope Entfernung, Strippen, Absorption, Pervaporation und Diffusion über Membranen oder Extraktion.

Das Strippen kann beispielsweise durch Durchleiten eines sauerstoffhaltigen Gases, bevorzugt eines Luft oder Luft-Stickstoff-Gemisches, durch das Reaktionsgemisch erfolgen, gegebenenfalls zusätzlich zu einer Destillation.

Zur Absorption eignen sich vorzugsweise Molekularsiebe oder Zeolithe (Porengröße z. B. im Bereich von etwa 3-10 Angström), eine Abtrennung durch Destillation oder mit Hilfe geeigneter semipermeabler Membranen.

Es ist aber auch möglich, das abgetrennte Gemisch aus (Meth)acrylsäureester (D) und dem diesem zugrundeliegenden Alkohol, das häufig ein Azeotrop bildet, direkt in eine Anlage zur Herstellung des (Meth)acrylsäureesters (D) zuzuführen, um es dort in einer Veresterung mit (Meth)acrylsäure wiederzuverwerten.

Nach Beendigung der Reaktion kann man das aus der Umesterung erhaltene Reaktionsgemisch ohne weitere Aufreinigung verwenden oder es erforderlichenfalls in einem weiteren Schritt aufreinigen.

In der Regel wird in einem Aufreinigungsschritt lediglich der eingesetzte Katalysator vom Reaktionsgemisch abgetrennt und das Reaktionsprodukt vom gegebenenfalls verwendeten organischen Lösungsmittel abgetrennt.

Eine Abtrennung von heterogenen Katalysatoren erfolgt in der Regel durch Filtration, Elektrofiltration, Absorption, Zentrifugation oder Dekantieren. Der abgetrennte heterogene Katalysator kann anschließend für weitere Reaktionen eingesetzt werden.

Bevorzugt werden im ersten Aufreinigungsschritt jedoch lediglich der Katalysator und das gegebenenfalls eingesetzte Lösungsmittel abgetrennt.

Das gegebenenfalls aufgereinigte Reaktionsgemisch wird gegebenenfalls einer Destillation unterworfen, in der der (Meth)acrylsäureester (F) der N,N-substituierten Aminoalkohole destillativ von unumgesetztem (Meth)acrylsäureester (D) sowie gegebenenfalls gebildeten Nebenprodukten getrennt wird.

Bei den Destillationseinheiten handelt es sich zumeist um Rektifikationskolonnen üblicher Bauart mit Umlaufverdampfer und Kondensator. Der Zulauf erfolgt in bevorzugt den Sumpfbereich, die Sumpftemperatur beträgt hier beispielsweise 80 bis 160 °C, bevorzugt 100 bis 140 °C, die Kopftemperatur bevorzugt 80 bis 120 °C und der Kopfdruck 3 bis 20, bevorzugt 3 bis 5 mbar. Selbstverständlich kann der Fachmann auch andere Temperatur- und Druckbereiche ermitteln, in denen die jeweiligen (Meth)acrylsäureester (F) der N,N-substituierten Aminoalkohole destillativ gereinigt werden können. Wesentlich ist dabei eine Trennung des Wunschproduktes von Edukten und Nebenprodukten unter Bedingungen, bei denen das Wunschprodukt möglichst keiner Abbaureaktion ausgesetzt ist.

Die Destillationseinheit weist in der Regel 5 bis 50 theoretische Böden auf.

Die Destillationseinheiten sind von an sich bekannter Bauart und weisen die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt.

Bevorzugt wird das Wunschprodukt diskontinuierlich destilliert, wobei zunächst Leichtsieder aus dem Reaktionsgemisch entfernt werden, zumeist Lösungsmittel oder unumgesetzter (Meth)acrylsäureester (D). Nach Abtrennung dieser Leichtsieder wird die Destillationstemperatur erhöht und/oder das Vakuum verringert und das Wunschprodukt abdestilliert.

Der verbleibende Destillationsrückstand wird zumeist verworfen.

Ferner kann das Endprodukt durch Adsorption gereinigt und erhalten werden, beispielsweise durch Adsorption mittels Aluminiumoxid, Aktivkohle, Silica oder andere dem Fachmann bekannte Adsorptionsmittel.

Die Reaktionsbedingungen bei der erfindungsgemäßen Umesterung sind mild. Aufgrund der niedrigen Temperaturen und sonstigen milden Bedingungen wird die Bildung von Nebenprodukten in der Reaktion vermieden, die andernfalls z.B. durch unerwünschte radikalische Polymerisation des eingesetzten (Meth)acrylsäureesters (D), die sonst nur durch Zugabe von Polymerisationsinhibitoren verhindert werden kann.

Bei der erfindungsgemäßen Reaktionsführung kann der Reaktionsmischung über den ohnehin in dem (Meth)acrylsäureester (D) enthaltenen Polymerisationsinhibitoren hinaus zusätzlicher Polymerisationsinhibitoren zugegeben werden, beispielsweise Hydrochinonmonomethylether, Phenothiazin, Phenole, wie z.B. 2-tert.-Butyl-4-methylphenol, 6-tert.-Butyl-2,4-dimethyl-phenol oder N-Oxyle, wie 4-Hydroxy-2,2,6,6-tetramethyl-piperidin-N-oxyl, 4-Oxo-2,2,6,6-tetramethylpiperidin-N-oxyl oder Uvinul^{®} 4040P der BASF Aktiengesellschaft, wobei erfindungsgemäß die Gesamtmenge von <450 ppm nicht überschritten werden darf. Vorteilhaft wird die Umesterung in Gegenwart eines sauerstoffhaltigen Gases, bevorzugt Luft oder Luft-Stickstoff-Gemische, durchgeführt.

Diese Polymerisationsinhibitoren können einzeln oder in beliebiger Mischung eingesetzt werden. Erfindungswesentlich ist jedoch, dass der Gehalt an Polymerisationsinibitor nicht mehr als 450 ppm, bevorzugt nicht mehr als 400 ppm, insbesondere nicht mehr als 380 ppm und besonders bevorzugt nicht mehr als 350 ppm beträgt. Dies ist insbesondere für die weitere Verwendung des (Meth)acrylsäureesters (F) von Bedeutung.

Die erfindungsgemäß hergestellten (Meth)acrylsäureester (F) von N,N-substituierten Aminoalkoholen (I) finden Anwendung beispielsweise als Monomere oder Comonomere in der Herstellung von Dispersionen, beispielsweise Acryldispersionen, als Reaktivverdünner, wie in strahlungshärtbaren Beschichtungsmassen oder in Farben, sowie in Dispersionen für Anwendung im Papierbereich, im Kosmetikbereich, im Pharmabereich, in Agroformulierungen, in der Textilindustrie und im Bereich der Ölförderung.

Die folgenden Beispiele sollen die Eigenschaften der Erfindung erläutern, ohne sie aber einzuschränken.

### Beispiele

Als "Teile" seien in dieser Schrift, wenn nicht anders angegeben, "Gewichtsteile" verstanden.

### Beispiel 1

Die Umesterung erfolgte in einem 750 mL Miniplantreaktor mit Oldershaw-Kolonne mit Flüssigkeitsverteiler, Innenthermometer, Gaseinleitung und Ankerrührer. Das Rücklaufverhältnis betrug 25 : 1 (Rücklauf: Ablauf), die Rührgeschwindigkeit des Anker-Rührers betrug 300 U/min und die Lufteinleitung 1,5 L/h.

In dieser Apparatur wurden 0,19 g (350 ppm) Hydrochinonmonomethylether, 600 g (6,0 mol) Methylmethacrylat (MMA) und 165,3 g (1,0 mol) N-Ethyl-N-hydroxyethylanilin sowie 4,25 g (2,0 mol-% bezogen auf N-Ethyl-N-hydroxyethylanilin) wasserfreies Kaliumphosphat vorgelegt und gerührt. Das Vakuum wurde eingestellt (300 mbar) und die Suspension wurde langsam auf 70 °C erhitzt. Während der Reaktion wurde kontinuierlich Destillat (MMA/Methanol) entfernt und entsprechend dem Rücklaufverhältnis zurückgeführt. Die Temperatur im Sumpf betrug zwischen 70 und 75 °C, die Temperatur am Kopf der Kolonne betrug zwischen 38 und 66°C. Die Reaktion wurde mittels GC-Analyse verfolgt. Nach 300 min wurde die Badtemperatur auf 60°C reduziert und nicht umgesetztes MMA abdestilliert. Die Reaktion wurde beendet und das Vakuum aufgehoben. Die Suspension wurde abgekühlt, und anschließend wurde über eine Druckfilternutsche der verbliebene Katalysator abfiltriert.

Man erhielt eine klare Lösung in einer Ausbeute von 90 % von Ethylanilinethylmethacrylat mit einer Reinheit von > 98 %.

### Beispiel 2

Die Umesterung erfolgte in der in Beispiel 1 beschriebenen Apparatur.

In dieser Apparatur wurden 0,30 g (350 ppm) Hydrochinonmonomethylether, 600 g (6,0 mol) Methylmethacrylat (MMA) und 259,9 g (1,5 mol) N,N-Dibutylaminoethanol sowie 6,37 g (2,0 mol-% bezogen auf N,N-Dibutylaminoethanol) wasserfreies Kaliumphosphat vorgelegt und gerührt. Das Vakuum wurde eingestellt (300 mbar) und die Suspension wurde langsam auf 70 °C erhitzt. Während der Reaktion wurde kontinuierlich Destillat (MMA/Methanol) entfernt und entsprechend dem Rücklaufverhältnis zurückgeführt. Die Temperatur im Sumpf betrug zwischen 70 und 76 °C, die Temperatur am Kopf der Kolonne betrug zwischen 38 und 66 °C. Die Reaktion wurde mittels GC-Analyse verfolgt. Nach 340 min wurde die Badtemperatur auf 60 °C reduziert und nicht umgesetztes MMA abdestilliert. Die Reaktion wurde beendet und das Vakuum aufgehoben. Die Suspension wurde abgekühlt, und anschließend wurde über eine Druckfilternutsche der verbliebene Katalysator abfiltriert.

Man erhielt eine klare Lösung in einer Ausbeute von 95 % von N,N-Dibutylaminoethylmethacrylat mit einer Reinheit von > 90 %.

### Beispiel 3

Die Umesterung erfolgte in der in Beispiel 1 beschriebenen Apparatur.

In dieser Apparatur wurden 0,25 g (350 ppm) Hydrochinonmonomethylether, 600 g (6,0 mol) Methylmethacrylat (MMA) und 219,5 g (1,5 mol) 2-(2-Dimethylamino-ethylamino)-ethanol sowie 6,37 g (2,0 mol-% bezogen auf 2-(2-Dimethylamino-ethylamino)-ethanol) wasserfreies Kaliumphosphat vorgelegt und gerührt. Das Vakuum wurde eingestellt (300 mbar) und die Suspension wurde langsam auf 70 °C erhitzt. Während der Reaktion wurde kontinuierlich Destillat (MMA/Methanol) entfernt und entsprechend dem Rücklaufverhältnis zurückgeführt. Die Temperatur im Sumpf betrug zwischen 70 und 76 °C, die Temperatur am Kopf der Kolonne betrug zwischen 38 und 66 °C. Die Reaktion wurde mittels GC-Analyse verfolgt. Nach 240 min wurde die Badtemperatur auf 60 °C reduziert und nicht umgesetztes MMA abdestilliert. Die Reaktion wurde beendet und das Vakuum aufgehoben. Die Suspension wurde abgekühlt, und anschließend wurde über eine Druckfilternutsche der verbliebene Katalysator abfiltriert.

Man erhielt eine klare Lösung in einer Ausbeute von 95 % von 2-(2-Dimethylamino-ethylamino)-ethylmethacrylat mit einer Reinheit von > 97 %.

### Beispiel 4

Die Umesterung erfolgte in der in Beispiel 1 beschriebenen Apparatur.

In dieser Apparatur wurden 0,28 g (350 ppm) Hydrochinonmonomethylether, 600 g (6,0 mol) Methylmethacrylat (MMA) und 193,8 g (1,5 mol) Hydroxyethylpiperidin sowie 6,37 g (2,0 mol-% bezogen auf Hydroxyethylpiperdin) wasserfreies Kaliumphosphat vorgelegt und gerührt. Das Vakuum wurde eingestellt (300 mbar) und die Suspension wurde langsam auf 70 °C erhitzt. Während der Reaktion wurde kontinuierlich Destillat (MMA/Methanol) entfernt und entsprechend dem Rücklaufverhältnis zurückgeführt. Die Temperatur im Sumpf betrug zwischen 70 und 76 °C, die Temperatur am Kopf der Kolonne betrug zwischen 38 und 66 °C. Die Reaktion wurde mittels GC-Analyse verfolgt. Nach 270 min wurde die Badtemperatur auf 60 °C reduziert und nicht umgesetztes MMA abdestilliert. Die Reaktion wurde beendet und das Vakuum aufgehoben. Die Suspension wurde abgekühlt, und anschließend wurde über eine Druckfilternutsche der verbliebene Katalysator abfiltriert.

Man erhielt eine klare Lösung in einer Ausbeute von > 99 % von Piperidinylethylmethacrylat mit einer Reinheit von ca. 90 %. Die Lösung enthielt noch 5 % des Alkohols.

Der verbliebene Alkohol wurde vom Produkt durch Destillation (0,1 mbar, 86 °C Sumpftemperatur) abgetrennt.

Das Endprodukt wurde in einer Ausbeute von 86 % und mit einer Reinheit von > 98 % erhalten.

### Beispiel 5

Die Umesterung erfolgte in der in Beispiel 1 beschriebenen Apparatur.

In dieser Apparatur wurden 0,28 g (350 ppm) Hydrochinonmonomethylether, 600 g (6,0 mol) Methylmethacrylat (MMA) und 196,7 g (1,5 mol) Hydroxyethylmorpholin sowie 6,37 g (2,0 mol-% bezogen auf Hydroxyethylmorpholin) wasserfreies Kaliumphosphat vorgelegt und gerührt. Das Vakuum wurde eingestellt (300 mbar) und die Suspension wurde langsam auf 70 °C erhitzt. Während der Reaktion wurde kontinuierlich Destillat (MMA/Methanol) entfernt und entsprechend dem Rücklaufverhältnis zurückgeführt. Die Temperatur im Sumpf betrug zwischen 70 und 76 °C, die Temperatur am Kopf der Kolonne betrug zwischen 38 und 66 °C. Die Reaktion wurde mittels GC-Analyse verfolgt. Nach 360 min wurde die Badtemperatur auf 60 °C reduziert und nicht umgesetztes MMA abdestilliert. Die Reaktion wurde beendet und das Vakuum aufgehoben. Die Suspension wurde abgekühlt, und anschließend wurde über eine Druckfilternutsche der verbliebene Katalysator abfiltriert.

Man erhielt eine klare Lösung in einer Ausbeute von 86,5 % von Morpholinoethylmethacrylat mit einer Reinheit von > 92 %. Die Lösung enthielt noch 3,7 % des Alkohols.

Der verbliebene Alkohol wurde vom Produkt durch Destillation (0,1 mbar, 86 °C Sumpftemperatur) abgetrennt.

Das Endprodukt wurde in einer Ausbeute von 86 % und mit einer Reinheit von > 98 % erhalten.

### Beispiel 6

Die Umesterung wurde in einem beheizbaren 4L Doppelmantelreaktor mit Ankerrührer, Innenthermometer, Gaseinleitung, Kolonne (strukturierte Sulzer CY Packung, 9 theoretische. Böden) und Flüssigkeitsteiler durchgeführt.

Es wurde eine Lösung von 1,06 g (350 ppm) Hydrochinonmonomethylether (MeHQ) in 2000 g (20 mol) Methylmethacrylat (MMA) vorgelegt. 1032 g (10 mol) Dimethylaminopropanol sowie 42,45 g wasserfreies Kaliumphosphat wurden hinzugegeben und unter Lufteinleitung (1,5 L/h) sowie einem Vakuum von 300 mbar bis zum Sieden erwärmt (95°C Manteltemperatur). Es wurde ein Rücklaufverhältnis von 10:1, dann 5:1 (nach 15 min), dann 2:1 (nach 60 min) und zuletzt wieder 5:1 (nach 90 min) eingestellt. Das entstehende Azeotrop aus Methanol sowie MMA wurde abdestilliert. Die Sumpftemperatur betrug während der Reaktion 70-90°C, die Kopftemperatur 33-63°C. Nach 360 min wurde auf 64°C abgekühlt und nicht umgesetztes MMA abdestilliert. Die Suspension wurde abgekühlt und über eine Druckfilternutsche filtriert.

Es wurden 1517 g des Produkts in einer Reinheit von 97 % erhalten.

## Patentansprüche

1. Verfahren zur Herstellung von (Meth)acrylsäureestern (F) von N,N-substituierten Aminoalkoholen, wobei man N,N-substituierte Aminoalkohole (I), 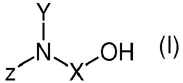 worin
Y und Z unabhängig voneinander C₁-C₂₀-Alkyl, C₃-C₁₅-Cycloalkyl, Aryl oder Y und Z zusammen mit dem sie verbindenden Stickstoffatom einen 5- bis 9-gliedrigen gesättigten heterocyclischen Rest bilden, der gegebenenfalls Sauerstoff, Schwefel, Stickstoff oder C₁-C₄-alkylsubstituierten Stickstoff als weiteres Heteroatom aufweist, und
X C₂-C₂₀-Alkylen, das durch 1 bis 10 nichtbenachbarte Oxy-, und/oder unsubstituierte oder mit Methoxy substituierte C₁-C₄-Alkyliminogruppen unterbrochen sein kann, oder C₃-C₁₅-Cycloalkylen
bedeuten,
in Gegenwart mindestens eines heterogenen Katalysators (K) mit mindestens einem (Meth)acrylsäureester (D) umestert, **dadurch gekennzeichnet, dass** der heterogene Katalysator (K) ein anorganisches Salz ist, ausgewählt aus der Gruppe bestehend aus Li₃PO₄, K₃PO₄, Na₃ PO₄ und K₂CO₃ sowie deren Hydrate und ohne weitere Lösungsmittel eingesetzt wird und der Gehalt an Polymerisationsinhibitoren in der Reaktionsmischung ≤ 450 ppm beträgt.

2. Verfahren gemäß Anspruch 1, **dadurch gekennzeichnet, dass** die Substituenten Y und Z jeweils unabhängig voneinander C₁-C₁₀-Alkyl, Aryl oder Y und Z zusammen mit dem sie verbindenden Stickstoffatom einen 5- bis 9-gliedrigen heterocyclischen Rest, der gegebenenfalls Sauerstoff, Stickstoff oder C₁-C₄-alkylsubstituierten Stickstoff als weiteres Heteroatom aufweist, bedeuten.

3. Verfahren gemäß Anspruch 2, **dadurch gekennzeichnet, dass** die Substituenten Y und Z jeweils unabhängig voneinander C₁-C₆-Alkyl oder Phenyl bedeuten.

4. Verfahren gemäß Anspruch 1, **dadurch gekennzeichnet, dass** Y und Z zusammen mit dem sie verbindenden Stickstoffatom einen 5- oder 6-gliedrigen heterocyclischen Rest, der gegebenenfalls Sauerstoff, Stickstoff oder C₁-C₄-alkylsubstituierten Stickstoff als weiteres Heteroatom aufweist, bedeuten.

5. Verfahren nach Anspruch 1, **dadurch gekennzeichnet, dass** der Substituent X C₂-C₁₀-Alkylen, das durch 1 oder 5 nichtbenachbarte Oxy- und/oder unsubstituierte oder mit Methoxy substituierte C₁-C₄-Alkyliminogruppen unterbrochen sein kann, bedeutet.

6. Verfahren gemäß einem der vorstehenden Ansprüche, **dadurch gekennzeichnet, dass** der N,N-substituierte Aminoalkohol (I) ausgewählt ist aus der Gruppe bestehend aus N,N-Dibutylaminoethanol, N,N-Dimethylaminopropanol, 2-[(2-Dimethylamino-ethyl)-methyl-amino]-ethanol, 2-(2-Dimethylamino-ethoxy)-ethanol, N,N-Diethylaminoethanol, Piperidylethanol, Morpholylethanol und Ethylanilinethanol.

7. Verfahren nach einem der vorstehenden Ansprüche, **dadurch gekennzeichnet, dass** es sich bei dem (Meth)acrylsäureester (D) um einen gesättigten C₁-C₁₀-Alkylester handelt.

8. Verfahren nach einem der vorstehenden Ansprüche, **dadurch gekennzeichnet, dass** der (Meth)acrylsäureester (D) ausgewählt ist aus der Gruppe bestehend aus (Meth)acrylsäuremethylester, (Meth)acrylsäureethylesterm (Meth)acrylsäure-n-butylester und (Meth)acrylsäure-2-ethylhexylester.

9. Verfahren nach einem der vorstehenden Ansprüche, **dadurch gekennzeichnet, dass** der Gehalt an Polymerisationsinhibitoren in der Reaktionsmischung < 350 ppm beträgt.

## Claims

1. A process for preparing (meth)acrylic esters (F) of N,N-substituted amino alcohols, by transesterifying N,N-substituted amino alcohols (I) 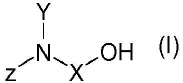 in which
Y and Z are each independently C₁-C₂₀-alkyl, C₃-C₁₅-cycloalkyl, aryl, or Y and Z together with the nitrogen atom connecting them form a 5-to 9-membered saturated heterocyclic radical which optionally has oxygen, sulfur, nitrogen or C₁-C₄-alkyl-substituted nitrogen as a further heteroatom, and
X is C₂-C₂₀-alkylene which may be interrupted by 1 to 10 nonadjacent oxy groups and/or unsubstituted or methoxy-substituted C₁-C₄-alkylimino groups, or a C₃-C₁₅-cycloalkylene,
with at least one (meth) acrylic ester (D) in the presence of at least one heterogeneous catalyst (K), wherein the heterogeneous catalyst (K) is an inorganic salt, selected from the group consisting of Li₃PO₄, K₃PO₄, Na₃PO₄ and K₂CO₃, and the hydrates thereof, and is used without any further solvent and the content of polymerization inhibitors in the reaction mixture is ≤ 450 ppm.

2. The process according to claim 1, wherein the substituents Y and Z are each independently C₁-C₁₀-alkyl, aryl, or Y and Z together with the nitrogen atom connecting them are a 5- to 9-membered heterocyclic radical which optionally has oxygen, nitrogen or C₁-C₄-alkyl-substituted nitrogen as a further heteroatom.

3. The process according to claim 2, wherein the substituents Y and Z are each independently C₁-C₆-alkyl or phenyl.

4. The process according to claim 1, wherein Y and Z together with the nitrogen atom connecting them are a 5- or 6-membered heterocyclic radical which optionally has oxygen, nitrogen or C₁-C₄-alkyl-substituted nitrogen as a further heteroatom.

5. The process according to claim 1, wherein the substituent X is C₂-C₁₀-alkylene which may be interrupted by 1 or 5 nonadjacent oxy groups and/or unsubstituted or methoxy-substituted C₁-C₄-alkylimino groups.

6. The process according to any of the preceding claims, wherein the N,N-substituted amino alcohol (I) is selected from the group consisting of N,N-dibutylaminoethanol, N,N-dimethylaminopropanol, 2-[(2-dimethylaminoethyl)methylamino]ethanol, 2-(2-dimethylaminoethoxy)ethanol, N,N-diethylaminoethanol, piperidylethanol, morpholylethanol and ethylanilineethanol.

7. The process according to any of the preceding claims, wherein the (meth)acrylic ester (D) is a saturated C₁-C₁₀-alkyl ester.

8. The process according to any of the preceding claims, wherein the (meth)acrylic ester (D) is selected from the group consisting of methyl (meth)acrylate, ethyl (meth)acrylate, n-butyl (meth)acrylate and 2-ethylhexyl (meth)acrylate.

9. The process according to any of the preceding claims, wherein the content of polymerization inhibitors in the reaction mixture is < 350 ppm.

## Revendications

1. Procédé de fabrication d'esters de l'acide (méth)acrylique (F) d'aminoalcools N,N-substitués, selon lequel des aminoalcools N,N-substitués (I) 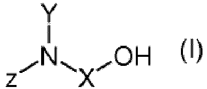 dans lesquels
Y et Z signifient indépendamment l'un de l'autre alkyle en C₁-C₂₀, cycloalkyle en C₃-C₁₅, aryle, ou Y et Z forment ensemble avec l'atome d'azote auquel ils sont reliés un radical hétérocyclique saturé de 5 à 9 éléments, qui comprend éventuellement un oxygène, un soufre, un azote ou un azote à substitution alkyle en C₁-C₄ en tant qu'hétéroatome supplémentaire, et
X signifie alkylène en C₂-C₂₀, qui peut être interrompu par 1 à 10 groupes oxy non voisins et/ou groupes alkylimino en C₁-C₄ non substitués ou substitués par méthoxy, ou cycloalkylène en C₃-C₁₅,
sont transestérifiés en présence d'au moins un catalyseur hétérogène (K) avec au moins un ester de l'acide (méth)acrylique (D), **caractérisé en ce que** le catalyseur hétérogène (K) est un sel inorganique choisi dans le groupe constitué par Li₃PO₄, K₃PO₄, Na₃PO₄ et K₂CO₃, ainsi que leurs hydrates, et est utilisé sans solvant supplémentaire, et la teneur en inhibiteurs de polymérisation dans le mélange réactionnel est ≤ 450 ppm.

2. Procédé selon la revendication 1, **caractérisé en ce que** les substituants Y et Z signifient chacun indépendamment l'un de l'autre alkyle en C₁-C₁₀, aryle, ou Y et Z forment ensemble avec l'atome d'azote auquel ils sont reliés un radical hétérocyclique de 5 à 9 éléments, qui comprend éventuellement un oxygène, un azote ou un azote à substitution alkyle en C₁-C₄ en tant qu'hétéroatome supplémentaire.

3. Procédé selon la revendication 2, **caractérisé en ce que** les substituants Y et Z signifient chacun indépendamment l'un de l'autre alkyle en C₁-C₆ ou phényle.

4. Procédé selon la revendication 1, **caractérisé en ce qu'**Y et Z forment ensemble avec l'atome d'azote auquel ils sont reliés un radical hétérocyclique à 5 ou 6 éléments, qui comprend éventuellement un oxygène, un azote ou un azote à substitution alkyle en C₁-C₄ en tant qu'hétéroatome supplémentaire.

5. Procédé selon la revendication 1, **caractérisé en ce que** le substituant X signifie alkylène en C₂-C₁₀, qui qui peut être interrompu par 1 ou 5 groupes oxy non voisins et/ou groupes alkylimino en C₁-C₄ non substitués ou substitués par méthoxy,

6. Procédé selon l'une quelconque des revendications précédentes, **caractérisé en ce que** l'aminoalcool N,N-substitué (I) est choisi dans le groupe constitué par le N,N-dibutylaminoéthanol, le N,N-diméthylaminopropanol, le 2-[(2-diméthylamino-éthyl)-méthyl-amino]-éthanol, le 2-(2-diméthylamino-éthoxy)-éthanol, le N,N-diéthylaminoéthanol, le pipéridyléthanol, le morpholyléthanol et l'éthylaniline-éthanol.

7. Procédé selon l'une quelconque des revendications précédentes, **caractérisé en ce que** l'ester de l'acide (méth) acrylique (D) est un ester alkylique en C₁-C₁₀ saturé.

8. Procédé selon l'une quelconque des revendications précédentes, **caractérisé en ce que** l'ester de l'acide (méth)acrylique (D) est choisi dans le groupe constitué par l'ester méthylique de l'acide (méth)acrylique, l'ester éthylique de l'acide (méth)acrylique, l'ester n-butylique de l'acide (méth)acrylique et l'ester 2-éthylhexylique de l'acide (méth)acrylique.

9. Procédé selon l'une quelconque des revendications précédentes, **caractérisé en ce que** la teneur en inhibiteurs de polymérisation dans le mélange réactionnel est < 350 ppm.
